# EUROPEAN PATENT APPLICATION

(11) **EP 3 872 176 A1**
(43) Date of publication of application: **01.09.2021**
(21) Application number: 19874976.4
(22) Date of filing: 17.10.2019
(51) Int. Cl.: C12N 11/12, C12N 9/96

(54) **CARRIER FOR ENZYME IMMOBILIZATION USE, AND IMMOBILIZED ENZYME**

(30) Priority: 24.10.2018 JP 2018200157
(71) Applicant: Tokyo Institute of Technology, Tokyo 152-8550 (JP); DKS Co. Ltd., Kyoto-shi, Kyoto 600-8873 (JP)
(72) Inventor: SERIZAWA Takeshi, Tokyo 152-8550 (JP); SAWADA Toshiki, Tokyo 152-8550 (JP); NISHIURA Masahito, Kyoto-shi, Kyoto 600-8873 (JP)
(74) Representative: Hoffmann Eitle
(86) International application number: PCT/JP2019/040990
(87) International publication number: WO 2020/085217

(57) **Abstract**

This invention provides a novel carrier for enzyme immobilization and an immobilized enzyme. The carrier for enzyme immobilization according to an embodiment comprises a porous material and cellulose that has an amino group-containing substituent at an anomeric position and is immobilized on the porous material. The immobilized enzyme contains the carrier for enzyme immobilization and an enzyme immobilization on the cellulose. The carrier for enzyme immobilization is obtained by adding an acid to an aqueous solution in which cellulose having an amino group-containing substituent at an anomeric position is dissolved in an aqueous alkaline solution to deposit the cellulose in the presence of a porous material. The immobilized enzyme is obtained by immobilizing an enzyme on the cellulose.

## Description

### Technical Field

The present invention relates to a carrier for enzyme immobilization, an immobilized enzyme, and a method for producing the carrier and the enzyme.

### Background Art

Methods for artificially synthesizing crystalline cellulose are known. For example, cellulose can be artificially synthesized by reacting α-glucose-1-phosphate (αG1P) and glucose as a primer with cellodextrin phosphorylase (CDP), which is a phosphorolytic enzyme, using the reverse reaction of CDP (see Patent Literature (PTL) 1).

In terms of the artificial synthesis of cellulose using CDP, it is also known to synthesize cellulose having a substituent by using a glucose derivative having a substituent at its anomeric position as a primer (see PTL 2).

Cellulose obtained by artificial synthesis is usually an oligomer due to its low degree of polymerization, and is also called "cellooligosaccharide" or "cellodextrin," but is referred to here as "cellulose" without differentiation.

### Citation List

### Patent Literature

PTL 1: WO2016/113933
PTL 2: WO2016/140369

### Summary of Invention

### Technical Problem

Conventionally, enzymes are immobilized on carriers to achieve more effective use of them.

An object of an embodiment of the present invention is to provide a novel carrier for enzyme immobilization that is capable of immobilizing an enzyme using artificially synthesized cellulose, provide an immobilized enzyme, and provide a method for producing the carrier and the enzyme.

### Solution to Problem

A carrier for enzyme immobilization according to an embodiment of the present invention comprises a porous material and cellulose, the cellulose having an amino group-containing substituent at an anomeric position and being immobilized on the porous material.

An immobilized enzyme according to an embodiment of the present invention comprises the carrier for enzyme immobilization and an enzyme immobilized on the cellulose.

A method for producing a carrier for enzyme immobilization according to an embodiment of the present invention comprises
adding an acid to an aqueous solution in which cellulose having an amino group-containing substituent at an anomeric position is dissolved in an aqueous alkaline solution to deposit the cellulose in the presence of a porous material to thereby immobilize the cellulose on the porous material.

A method for producing an immobilized enzyme according to an embodiment of the present invention comprises
adding an acid to an aqueous solution in which cellulose having an amino group-containing substituent at an anomeric position is dissolved in an aqueous alkaline solution to deposit the cellulose in the presence of a porous material to thereby immobilize the cellulose on the porous material, and
immobilizing an enzyme on the cellulose.

### Advantageous Effects of Invention

Embodiments of the present invention can provide a carrier for enzyme immobilization that is capable of easily immobilizing an enzyme, and provide an immobilized enzyme.

### Brief Description of Drawings

Fig. 1 is graphs showing the ultraviolet-visible absorption spectra of liquids used for washing composites, showing the results of evaluation for stability of the composites of Examples 1 and 2 and Comparative Examples 1 to 6.
Fig. 2 is a graph showing the infrared absorption spectra of the composites of Example 2 and Comparative Example 6.
Fig. 3 is a graph showing the ultraviolet-visible absorption spectra of reaction liquids, showing the enzyme activities in Example 2 and Comparative Example 6.
Fig. 4 is a graph showing the absorbance at 405 nm in the ultraviolet-visible absorption spectra of reaction liquids, showing the enzyme activities in Example 2 and Comparative Example 6.

### Description of Embodiments

### 1. Carrier for Enzyme Immobilization

The carrier for enzyme immobilization according to this embodiment comprises (A) a porous material and (B) cellulose having an amino group (sometimes referred to below as the "aminated cellulose").

### (A) Porous Material

A porous material is a substance with many micropores, and various water-insoluble porous materials can be used as a carrier. The porous material may be fibrous or non-fibrous. The porous material is preferably a material into which water can be impregnated, and more preferably a material that has through pores that are water permeable.

Examples of fibrous materials (i.e., porous fibrous materials) include paper such as filter paper, and nonwoven fabric, woven fabric, knitted fabric, and glass-fiber filter paper. The filter paper may be cellulose filter paper formed of plant fibers. Examples of the materials of nonwoven, woven, and knitted fabrics include polypropylene fibers, polyester fibers, polyamide fibers, and carbon fibers.

Examples of the materials of non-fibrous materials (i.e., porous non-fibrous materials) include inorganic materials such as ceramics, glass, and metals; and organic materials such as thermoplastic resin and thermosetting resin.

### (B) Aminated Cellulose

Aminated cellulose for use refers to cellulose that has a structure in which glucose units are linked through β-1,4 linkages (more specifically, cellooligosaccharide), and has an amino group-containing substituent at an anomeric position. The amino group is preferably a primary amino group. The presence of amino group enables adsorption and immobilization of an enzyme.

More specifically, the aminated cellulose for use is preferably a compound represented by the following formula (1): 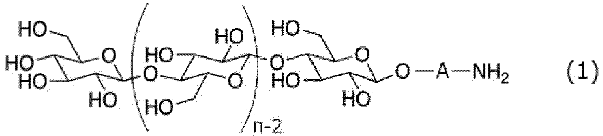 wherein A represents a divalent hydrocarbon group having 1 to 20 carbon atoms, and n is 6 to 16.

A in the formula may be a divalent aliphatic hydrocarbon group or a divalent aromatic hydrocarbon group. Examples of divalent aliphatic hydrocarbon groups include alkanediyl groups and alkenediyl groups, which may be linear or branched. Examples of divalent aromatic hydrocarbon groups include divalent aliphatic hydrocarbon groups having aromatic ring substituents, and arenediyl groups, with these aromatic rings being optionally substituted with, for example, alkyl. The number of carbon atoms in A is preferably 1 to 10, and more preferably 1 to 5. A is preferably an alkanediyl group having 1 to 10 carbon atoms, and more preferably an alkanediyl group having 1 to 5 carbon atoms.

n in the formula represents the degree of polymerization of cellulose, and is an integer of 6 to 16. n is preferably 7 or more and is preferably 15 or less. The aminated cellulose represented by formula (1) is usually a mixture of compounds with different degrees of polymerization. The average degree of polymerization is not particularly limited, and is preferably, for example, 7 or more or 8 or more, and is preferably, for example, 14 or less or 12 or less.

In one embodiment, the aminated cellulose for use is preferably represented by the following formula (2): 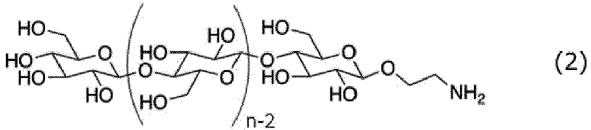 wherein n is as defined for formula (1).

The aminated cellulose according to this embodiment may be a cellulose structure having cellulose type-II crystalline structure. That is, in one embodiment, the aminated cellulose may be a cellulose structure having cellulose type-II crystalline structure and containing as a constituent component a compound represented by formula (1). The cellulose structure may have a sheet-like structure (cellulose nanosheet). Unlike naturally occurring cellulose chains, which have cellulose type-I crystalline structure (the chains being arranged in parallel), artificially synthesized aminated cellulose forms a cellulose type-II crystalline structure, which is thermodynamically stable. At this time, the amino group-containing substituent at an end of the cellulose chain (e.g., 2-aminoethyl group in formula (2)) does not affect the crystal form. The cellulose derivatives having an amino group-containing substituent are arranged in the thickness direction of the cellulose nanosheet to form lamellar crystals, and the amino groups are exposed on the sheet surface.

The method for synthesizing aminated cellulose according to this embodiment is not particularly limited. For example, the aminated cellulose represented by formula (2) can be produced according to the following reaction.

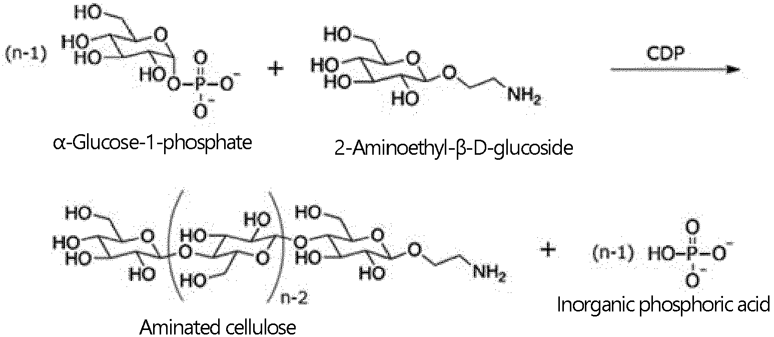

This reaction is an enzymatic synthesis reaction using the reverse reaction of cellodextrin phosphorylase (CDP). When α-glucose-1-phosphate (αG1P) and 2-aminoethyl-β-D-glucoside are reacted with CDP, αG1P as a monomer is sequentially polymerized onto 2-aminoethyl-β-D-glucoside as a primer, thus obtaining the aminated cellulose represented by formula (2). By changing the substituent at the anomeric position in the primer, aminated celluloses with various substituents represented by formula (1) can be synthesized.

2-Aminoethyl-β-D-glucoside can be synthesized with reference to the method described in, for example, B. Rao et al., Chem. Commun., 2013, 49, 10808-10810. More specifically, O-tetraacetyl-2-bromoethyl-β-D-glucoside is synthesized from O-pentaacetyl-β-D-glucoside and 2-bromoethanol, and O-pentaacetyl-2-azidoethyl-β-D-glucoside is synthesized from O-pentaacetyl-2-bromoethyl-β-D-glucoside and sodium azide, followed by deacetylation with sodium methoxide. Subsequently, the azide group is reduced, whereby 2-aminoethyl-β-D-glucoside can be obtained.

CDP is known to be produced from microorganisms, such as *Clostridium thermocellum* and *Cellulomonas sp.* CDP can be obtained by using known methods using these microorganisms. For example, CDP from *Clostridium thermocellum* YM4 can be prepared by an *E. coli* expression system according to the method described in M. Krishnareddy et al., J. Appl. Glycosci., 2002, 49, 1-8. However, the method is not limited to this.

The concentration of CDP is not particularly limited and may be, for example, 0.1 U/ml or more or 0.2 U/ml or more. The enzyme amount of CDP can be determined, for example, based on the enzyme activity. In this case, for example, αG1P, D-(+)-cellobiose, and CDP are incubated, the phosphoric acid produced from CDP is quantified, and the amount of enzyme that liberates 1 µmol of phosphoric acid per minute can be defined as 1 U.

For example, 10 to 1000 mM αG1P, 10 to 200 mM 2-aminoethyl-β-D-glucoside, and 0.1 U/mL or more of CDP are mixed in 100 to 1000 mM 2-[4-(2-hydroxyethyl)-1-piperazinyl]ethanesulfonic acid (HEPES) buffer (pH of 7.0 to 8.0), and the mixture is incubated at 10 to 80°C for 30 minutes to 30 days to allow a reaction to proceed, whereby the aminated cellulose of formula (2) can be synthesized.

In the carrier for enzyme immobilization according to this embodiment, aminated cellulose is immobilized on a porous material. Specifically, the aminated cellulose is retained within the porous material so as to be prevented from being removed even when washed with water. As described above, aminated cellulose, which has an amino group, can immobilize an enzyme; thus, an enzyme can be immobilized on a porous material through the aminated cellulose.

The ratio of immobilized aminated cellulose to the porous material is not particularly limited. For example, the amount of aminated cellulose attached may be 0.1 to 30 parts by mass, or may be 1 to 20 parts by mass, per 100 parts by mass of the porous material.

### 2. Immobilized Enzyme

An immobilized enzyme according to this embodiment comprises the carrier for enzyme immobilization described above and an enzyme immobilized on aminated cellulose. That is, the immobilized enzyme comprises a porous material, aminated cellulose immobilized on the porous material, and an enzyme immobilized on the aminated cellulose.

The aminated cellulose is capable of adsorbing enzymes, which are proteins, while maintaining the activity of the enzymes. More specifically, an enzyme is monolayer-adsorbed on the surface of the cellulose structure of the aminated cellulose. The electrostatic interaction contributes to this adsorption.

Examples of enzymes for use include, but are not particularly limited to, various enzymes, such as oxidoreductases, transferases, hydrolases, lyases, isomerases, and ligases. In terms of adsorptive properties on the aminated cellulose, the enzyme preferably has an isoelectric point at a pH lower than the pH at which aminated cellulose is positively charged. In particular, it is preferable to use an enzyme that is negatively charged in the neutral range (e.g., pH of 6.5 to 7.5), and it is more preferable to use an enzyme that has an isoelectric point at a pH of 7.0 or lower. The isoelectric point is measured by isoelectric focusing, in which the enzyme is electrophoresed in a gel with a pH gradient, and the pH at the site in the gel in which migration ceases is defined as the isoelectric point.

The ratio of the immobilized enzyme to aminated cellulose is not particularly limited. For example, the amount of enzyme attached may be 0.1 to 20 parts by mass, or may be 1 to 10 parts by mass, per 100 parts by mass of the aminated cellulose.

### 3. Method for Producing Carrier for Enzyme Immobilization

The carrier for enzyme immobilization according to this embodiment can be produced by adding an acid to an aqueous solution in which the aminated cellulose is dissolved in an aqueous alkaline solution to deposit the aminated cellulose in the presence of a porous material. That is, when the aminated cellulose is deposited in the presence of a porous material, the aminated cellulose is immobilized on the porous material, thus obtaining a carrier for enzyme immobilization according to this embodiment.

The aqueous alkaline solution is not particularly limited as long as it can dissolve aminated cellulose, and may be an aqueous sodium hydroxide solution, an aqueous potassium hydroxide solution, or the like. The concentration of aminated cellulose in the aqueous solution in which the aminated cellulose is dissolved (referred to below as "the aqueous cellulose solution") is not particularly limited. For example, the concentration at the time when an acid is added to deposit the aminated cellulose may be 1 to 10% (w/v) or may be 1 to 5% (w/v). "% (w/v)" means the mass (g) of the target component contained in 100 mL of the solution.

When an acid is added to an alkaline aqueous cellulose solution to make the solution neutral or acidic, the aminated cellulose becomes insoluble and is deposited. At this time, incubation may be performed with stirring, vibration, or the like. Alternatively, incubation may be performed while allowing it to stand (i.e., without stirring or vibration). Incubation causes the aminated cellulose to self-assemble. That is, the aminated cellulose molecules are arranged to form a cellulose structure having cellulose type-II crystalline structure. The aminated cellulose self-assembles even when it is stirred or vibrated; however, when incubation is performed while allowing it to stand, the aminated cellulose can self-assemble into larger aggregates. The incubation here is not particularly limited. For example, incubation may be performed at 10 to 80°C for 30 minutes to 30 days.

When the aminated cellulose is deposited in the presence of a porous material during the incubation described above, the aminated cellulose is deposited on the porous material. Accordingly, the aminated cellulose is retained or entangled in the micropores of the porous material and is thus immobilized on the porous material.

Examples of specific methods for immobilizing aminated cellulose on a porous material include a method comprising adding an acid to the aqueous cellulose solution to make the solution neutral, and then immediately impregnating the neutralized aqueous cellulose solution into the porous material, followed by incubation in this state (method 1), and a method comprising impregnating the aqueous cellulose solution into a porous material, and then adding an acid to the aqueous cellulose solution to make the solution neutral, followed by incubation (method 2). The aqueous cellulose solution may be impregnated into the porous material, for example, by adding the aqueous cellulose solution to the porous material, or by immersing the porous material in the aqueous cellulose solution.

Both method 1 and method 2 above cause deposition of aminated cellulose in the presence of a porous material. In method 1, however, neutralization is performed first, and the solution is thus impregnated into the porous material with the aminated cellulose being partially deposited (i.e., self-assembled), which presumably makes impregnation of the aminated cellulose into the porous material difficult. For this reason, it is more preferable to first impregnate the aqueous cellulose solution into the porous material, and then neutralize the solution by adding an acid, as in method 2.

### 4. Method for Producing Immobilized Enzyme

The immobilized enzyme according to this embodiment can be produced by the following steps:
(1) a cellulose immobilization step of adding an acid to an aqueous solution in which the aminated cellulose is dissolved in an aqueous alkaline solution to deposit the aminated cellulose in the presence of a porous material to thus immobilize the aminated cellulose on the porous material; and
(2) an enzyme immobilization step of immobilizing an enzyme on the aminated cellulose.

Either the cellulose immobilization step or the enzyme immobilization step can be performed first. Specifically, after aminated cellulose is immobilized on a porous material, an enzyme may be immobilized on the aminated cellulose. Alternatively, after an enzyme is immobilized on aminated cellulose, the aminated cellulose on which the enzyme is immobilized may be immobilized on a porous material. In the cellulose immobilization step, the aminated cellulose is dissolved in an aqueous alkaline solution, which may undesirably cause denaturation of the enzyme. Therefore, the former method is preferable; i.e., it is preferable to first immobilize aminated cellulose on a porous material, and then immobilize an enzyme on the aminated cellulose.

The cellulose immobilization step can be performed in the same manner as in the method for producing a carrier for enzyme immobilization described above.

The specific method for the enzyme immobilization step is not particularly limited. Aminated cellulose can adsorb and immobilize an enzyme due to the presence of an amino group; thus, bringing an enzyme into contact with aminated cellulose should be sufficient. For example, a porous material on which aminated cellulose is immobilized may be impregnated with an aqueous solution of an enzyme, and incubation may be performed in this state, such as at 10 to 80°C for 30 minutes to 30 days. The aqueous solution of an enzyme for use is preferably a buffer solution whose pH is adjusted to a neutral range. Thereafter, washing is performed with a buffer solution or water that does not contain an enzyme, whereby an immobilized enzyme in which the enzyme is immobilized on the porous material via the aminated cellulose is obtained.

### 5. Use

The carrier for enzyme immobilization and the immobilized enzyme according to this embodiment may be used for various known applications, including biosensors, which use enzymes to, for example, detect substances, and bioreactors, which use enzymes to, for example, produce substances.

Further, the use of the carrier for enzyme immobilization according to this embodiment enables immobilization of an enzyme with simple operation of impregnating an aqueous solution of the enzyme, thus enabling a user to easily immobilize various enzymes to produce immobilized enzymes according to purpose.

### Examples

The present invention is described in more detail below with reference to Examples; however, the present invention is by no means limited to these Examples.

### 1. Reagent

4N aqueous sodium hydroxide solution, 6N hydrochloric acid, magnesium chloride hexahydrate, and 2-mercaptoethanol were purchased from Nacalai Tesque, Inc. αGDP disodium hydrate, β-galactosidase (β-Gal, isoelectric point: pH of 4.6), p-nitrophenyl β-D-galactopyranoside (PNPG), and sodium dihydrogen phosphate dihydrate were purchased from Wako Pure Chemical Industries, Ltd. Filter paper (Whatman, grade 1, circles, diameter: 10 mm) was purchased from GE Healthcare. Ultrapure water was supplied by a Milli-Q system (Milli-Q Advantage A-10, Merck Millipore). In addition, reagents of special grade or higher grade were purchased from Nacalai Tesque, Inc. and used.

### 2. Experimental Method

### (1) Preparation of Cellulose and Aminated Cellulose

Cellulose (formula (3) below) with an average degree of polymerization of 10 was synthesized according to the method described in T. Serizawa et al., Polym. J., 2016, 48, 539-544. Additionally, aminated cellulose (formula (4) below) with an average degree of polymerization of 10 was synthesized according to the following method.

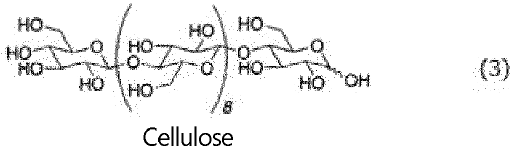

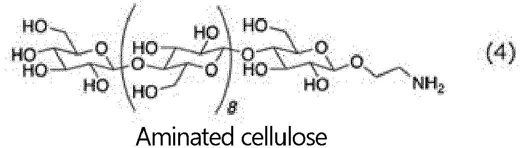

### Method for Synthesizing Aminated Cellulose

2-Aminoethyl-β-D-glucoside was prepared according to the method described in B. Rao et al., Chem. Commun., 2013, 49, 10808-10810. CDP was prepared according to the method described in M. Krishnareddy et al., J. Appl. Glycosci., 2002, 49, 1-8.

200 mM αG1P, 50 mM 2-aminoethyl-β-D-glucoside, and 0.2 U/mL CDP were mixed in 500 mM HEPES buffer (pH of 7.5), followed by incubation at 60°C for 3 days. The reaction liquid containing the precipitated product was centrifuged (15,000 rpm for 10 min or more at 4°C), the supernatant was removed, and then ultrapure water was added to redisperse the product, followed by centrifugation (the same conditions). This process was repeated to perform purification until the substitution percentage of the supernatant was 99.999% or more, thus obtaining aminated cellulose.

The average degree of polymerization of the product was measured using proton nuclear magnetic resonance (NMR) spectroscopy. Samples were prepared by dissolving 15 mg or more of the product that was lyophilized in 500 µL of 4% sodium deuteroxide solution. For the NMR spectrometer, an AVANCE III HD500 (Bruker Biospin, magnetic field strength: 500 MHz, integrated number: 16) was used. The average degree of polymerization was calculated based on the integrated values of protons in the anomeric position (δ: ∼4.2) at the reducing end and in the other anomeric positions (δ: ∼4.3) in the cellulose moiety of aminated cellulose.

### (2) Preparation of Filter Paper-Cellulose Composite and Filter Paper-Aminated Cellulose Composite

A predetermined amount of lyophilized cellulose or aminated cellulose was dispersed in a 1N aqueous sodium hydroxide solution in a 1.7-mL tube. The resulting product was incubated at -20°C for 30 minutes. After the temperature was returned to room temperature, the solution was dissolved by pipetting to prepare 2% (w/v), 4% (w/v), and 6% (w/v) aqueous solutions of cellulose, and a 6% (w/v) aqueous solution of aminated cellulose.

The immobilization of cellulose or aminated cellulose on filter paper, which is a porous material (i.e., obtaining composites of filter paper and cellulose or aminated cellulose) was performed based on two different methods of obtaining a composite.

In the first method of obtaining a composite (method 1), 15 µL each of the aqueous solutions of cellulose or aminated cellulose and 15 µL of 1N hydrochloric acid were mixed by pipetting in a 1.7-mL tube. Subsequently, the mixtures were immediately impregnated into filter paper placed in the wells of a 24-well plate and were then allowed to stand at 25°C for 1 hour. The composite obtained by using the 2% (w/v) aqueous solution of cellulose (final concentration of cellulose: 1% (w/v)) was used as Comparative Example 1, the composite obtained by using the 4% (w/v) aqueous solution of cellulose (final concentration of cellulose: 2% (w/v)) was used as Comparative Example 3, and the composite obtained by using the 6% (w/v) aqueous solution of cellulose (final concentration of cellulose: 3% (w/v)) was used as Comparative Example 5. The composite obtained by using the 6% (w/v) aqueous solution of aminated cellulose (final concentration of aminated cellulose: 3% (w/v)) was used as Example 1.

In the second method of obtaining a composite (method 2), filter paper was impregnated with 15 µL of the aqueous solutions of cellulose or aminated cellulose. Thereafter, 15 µL of 1N hydrochloric acid was impregnated into each piece of filter paper, and the resulting products were allowed to stand at 25°C for 1 hour. The composite obtained by using the 2% (w/v) aqueous solution of cellulose (final concentration of cellulose: 1% (w/v)) was used as Comparative Example 2, the composite obtained by using the 4% (w/v) aqueous solution of cellulose (final concentration of cellulose: 2% (w/v)) was used as Comparative Example 4, and the composite obtained by using the 6% (w/v) aqueous solution of cellulose (final concentration of cellulose: 3% (w/v)) was used as Comparative Example 6. The composite obtained by using the 6% (w/v) aqueous solution of aminated cellulose (final concentration of aminated cellulose: 3% (w/v)) was used as Example 2.

### (3) Evaluation for Stability of Filter Paper-Cellulose Composite and Filter Paper-Aminated Cellulose Composite

The filter paper-aminated cellulose composites of Examples 1 and 2 and the filter paper-cellulose composites of Comparative Examples 1 to 6 prepared by using methods 1 and 2 were each washed by pipetting 10 times with 3 mL of ultrapure water in a 24-well plate. The ultraviolet-visible absorption spectra of the liquids used for washing were measured by ultraviolet-visible spectrophotometer (V-670, JASCO Corporation). The measurement conditions were as follows: measurement wavelength: 200-800 nm, operation speed: 400 nm/min, bandwidth: 0.5 nm, data acquisition interval: 0.5 nm, response: fast.

At this time, the same measurement was performed for samples (control dispersions) obtained by dispersing in 3 mL of ultrapure water a product obtained by adding 15 µL of 1N hydrochloric acid to 15 µL each of the 2%, 4%, and 6% (w/v) aqueous solutions of cellulose and the 6% (w/v) aqueous solution of aminated cellulose.

The loading percentage of cellulose or aminated cellulose on the filter paper was calculated based on the absorbances at 500 nm (loading percentage (%) = 100 - 100 × absorbance of the washing liquid/absorbance of the control dispersion). Each experiment was conducted three times, and the average of the results of the three times was calculated.

### (4) Analysis of Crystal Structure of Cellulose and Aminated Cellulose Loaded on Filter Paper

The infrared absorption spectra of the filter paper-cellulose composite of Comparative Example 6 and the filter paper-aminated cellulose composite of Example 2 prepared by using method 2 were measured using attenuated total reflection infrared spectroscopy (FT/IR-4100 type A, JASCO Corporation) to evaluate the crystal structure of the cellulose and aminated cellulose loaded on the filter paper. Crystallized products of cellulose or aminated cellulose prepared beforehand by enzymatic reaction (control samples) and untreated filter paper alone were also measured in the same manner. The measurement conditions were as follows: measurement wavelength: 350-7800 cm⁻¹, resolution: 2 cm⁻¹, integrated number: 100.

### (5) Immobilization of Enzyme (β-Gal) on the Filter Paper-Cellulose Composite and the Filter Paper-Aminated Cellulose Composite, and Measurement of Its Activity

After 500 µL of β-Gal aqueous solution (10 nM β-Gal, 50 mM phosphate buffer, 1 mM magnesium chloride (pH of 7.3)) was added to the wells of a 24-well plate, the samples were immersed at 25°C for 30 min. Thereafter, the samples on which β-Gal was immobilized were transferred to a 24-well plate to which 1 mL of washing liquid (50 mM phosphate buffer, 1 mM magnesium chloride (pH of 7.3)) had been added, and allowed to stand for 15 minutes. This process was repeated 5 times to wash the samples. The filter paper-cellulose composite of Comparative Example 6, the filter paper-aminated cellulose composite of Example 2, and filter paper alone (filter paper moistened with 30 µL of ultrapure water) were used as the samples.

After 500 µL of an aqueous solution of PNPG, which is the substrate of β-Gal (1 mM PNPG, 86 mM phosphate buffer (pH of 7.3), 116 mM 2-mercaptoethanol, 1 mM magnesium chloride) was added to the wells of a 24-well plate, the samples on which β-Gal was immobilized were immersed at 25°C for 30 min to allow a reaction to proceed. The absorbance of each reaction liquid was measured with an ultraviolet-visible spectrophotometer (V-670, JASCO Corporation). The measurement conditions were as follows: measurement wavelength: 200-800 nm, operation speed: 400 nm/min, bandwidth: 0.5 nm, data acquisition interval: 0.5 nm, response: fast. Each experiment was conducted three times, and the average of the results of the three times was calculated.

### 3. Results and Discussion

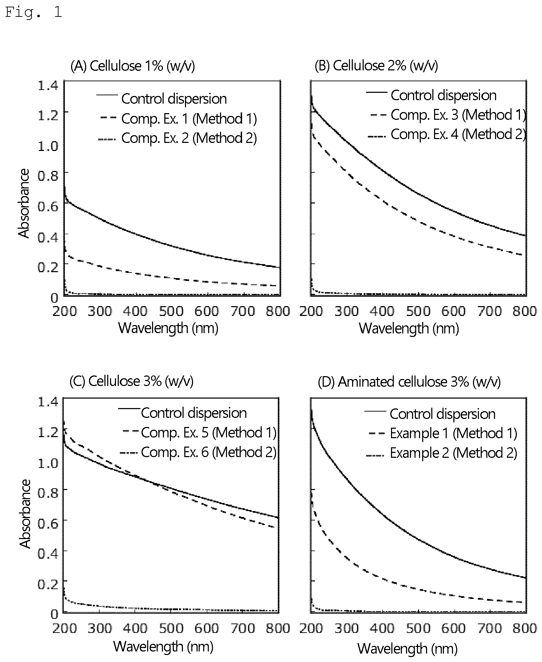

Fig. 1 shows the ultraviolet-visible absorption spectra of the liquids used for washing the composites in the evaluation of stability above. Table 1 below show the loading percentages obtained based on these results. With respect to the methods of obtaining a composite, loading percentages obtained in method 2 were higher than those obtained in method 1. The loading percentages obtained in method 2 were 97% or higher regardless of the conditions. In method 1, cellulose or aminated cellulose is allowed to partially self-assemble in advance; therefore, it is presumed that the self-assembled product formed in the solution is not easily impregnated into the filter paper, and is washed off from the filter paper. On the other hand, in method 2, the cellulose or aminated cellulose is allowed to self-assemble in the filter paper from the beginning; therefore, it is presumed that the self-assembled product is physically entangled with the mesh of the filter paper and is not easily washed off. These results revealed that, in order to stably load cellulose or aminated cellulose on the filter paper, the method in which cellulose or aminated cellulose is allowed to self-assemble in the filter paper from the beginning (method 2) is effective. However, even in method 1, aminated cellulose achieved 70% or higher immobilization. Therefore, as a method for producing a carrier for enzyme immobilization to immobilize aminated cellulose, method 1 according to Example 1 was also considered to be useful, as well as method 2 according to Example 2.

**Table 1**

| | Comp. Ex. 1 | Comp. Ex. 2 | Comp. Ex. 3 | Comp. Ex. 4 | Comp. Ex. 5 | Comp. Ex. 6 | Ex. 1 | Ex. 2 |
|---|---|---|---|---|---|---|---|---|
| Type and concentration of treatment liquid | Cellulose 1% (w/v) | | Cellulose 2%(w/v) | | Cellulose 3% (w/v) | | Aminated cellulose 3% (w/v) | |
| Method of obtaining a composite | Method 1 | Method 2 | Method 1 | Method 2 | Method 1 | Method 2 | Method 1 | Method 2 |
| Loading percentage (%) | 68.4 | 99.6 | 27.6 | 99.1 | 3.6 | 97.3 | 70.8 | 99.8 |

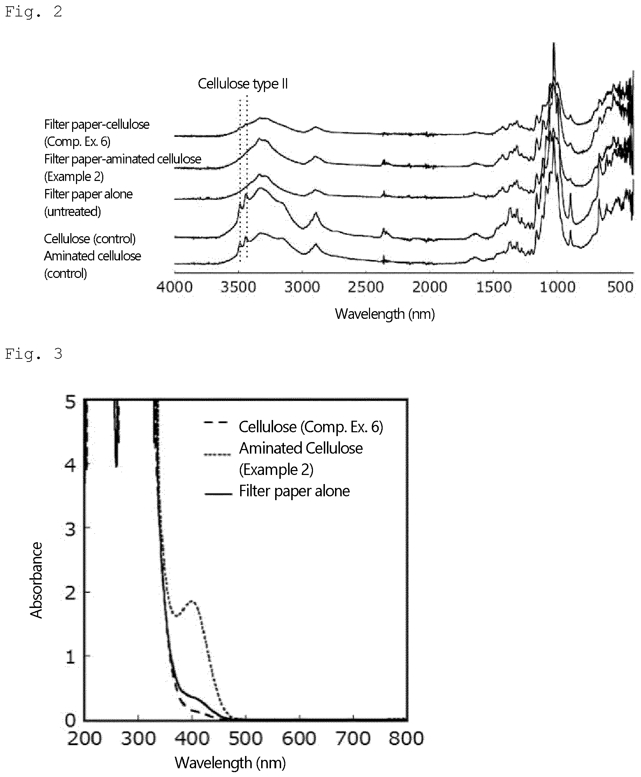

Fig. 2 shows the infrared absorption spectra of the filter paper-aminated cellulose composite of Example 2 and the filter paper-cellulose composite of Comparative Example 6. Two peaks indicating the presence of crystals of cellulose type II were observed around 3443 cm⁻¹ and 3491 cm⁻¹. Since these peaks were not observed in the filter paper alone, it was revealed that the cellulose or aminated cellulose self-assembled in the filter paper had cellulose type-II crystalline structure as in the control sample prepared beforehand by the enzymatic reaction.

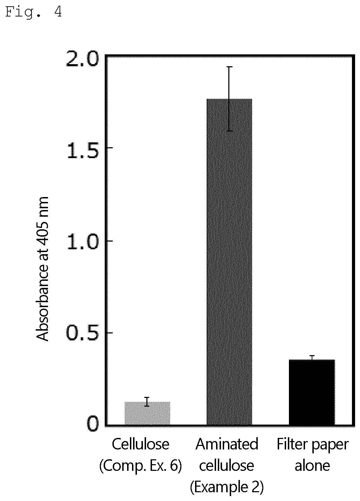

Fig. 3 shows the ultraviolet-visible absorption spectra of the reaction liquids in the measurement of enzyme activity above. In Example 2, in which β-Gal was immobilized on the filter paper-aminated cellulose composite, the absorption from p-nitrophenol generated when PNPG was hydrolyzed by β-Gal was observed around 400 nm, indicating that β-Gal immobilized on the filter paper through the aminated cellulose had enzymatic activity. A comparison of the absorbances at 405 nm revealed that, as shown in Fig. 4, Example 2, in which β-Gal was immobilized on the filter paper-aminated cellulose composite, showed 13.5-fold higher enzyme activity than that of Comparative Example 6, in which β-Gal was immobilized on the filter paper-cellulose composite. The enzyme activity of Example 2 was 4.9-fold higher than the case in which β-Gal was immobilized on the filter paper alone. These results revealed that when the filter paper on which aminated cellulose was loaded was used, an immobilized enzyme in which β-Gal was efficiently immobilized could be obtained while achieving the enzyme activity.

Some embodiments of the present invention are described above. However, these embodiments are presented as examples and are not intended to limit the scope of the invention. These embodiments can be embodied in a variety of other forms. Further, various omissions, substitutions, and changes can be made without departing from the spirit of the inventions. These embodiments, as well as, e.g., omissions of them, and substitutions or changes to them, are included in the scope and gist of the present invention, as well as in the scope of the invention recited in the claims and its equivalents.

## Claims

1. A carrier for enzyme immobilization comprising a porous material and cellulose, the cellulose having an amino group-containing substituent at an anomeric position and being immobilized on the porous material.

2. The carrier for enzyme immobilization according to claim 1, wherein the cellulose is represented by the following formula (1): 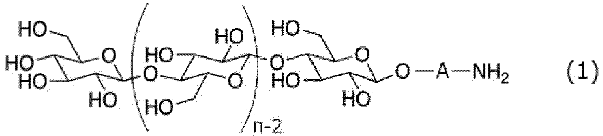 wherein A represents a divalent hydrocarbon group having 1 to 20 carbon atoms, and n is 6 to 16.

3. The carrier for enzyme immobilization according to claim 1 or 2, wherein the porous material comprises a fibrous material.

4. An immobilized enzyme comprising the carrier for enzyme immobilization of any one of claims 1 to 3, and an enzyme that is immobilized on the cellulose.

5. A method for producing a carrier for enzyme immobilization, comprising
adding an acid to an aqueous solution in which cellulose having an amino group-containing substituent at an anomeric position is dissolved in an aqueous alkaline solution to deposit the cellulose in the presence of a porous material to thereby immobilize the cellulose on the porous material.

6. A method for producing an immobilized enzyme, comprising
adding an acid to an aqueous solution in which cellulose having an amino group-containing substituent at an anomeric position is dissolved in an aqueous alkaline solution to deposit the cellulose in the presence of a porous material to thereby immobilize the cellulose on the porous material, and
immobilizing an enzyme on the cellulose.

7. The method for producing an immobilized enzyme according to claim 6, wherein the enzyme is immobilized on the cellulose after the cellulose is immobilized on the porous material.
